## Europäisches Patentamt
## European Patent Office
## Office européen des brevets

(19)

(11) Veröffentlichungsnummer: **0 215 858**
**B1**

(12) **EUROPÄISCHE PATENTSCHRIFT**

(45) Veröffentlichungstag der Patentschrift:
**13.09.89**

(51) Int. Cl.⁴: **A 61 H 7/00**

(21) Anmeldenummer: **86901779.8**

(22) Anmeldetag: **04.03.86**

(86) Internationale Anmeldenummer:
**PCT/DE 86/00083**

(87) Internationale Veröffentlichungsnummer:
**WO 86/05091 (12.09.86 Gazette 86/20)**

(54) **RÜCKENSTÜTZE ZUR PHYSIKALISCHEN THERAPIE VON ERKRANKUNGEN DER WIRBELSÄULE.**

(30) Priorität: **05.03.85 DE 3507746**
**15.10.85 DE 3536644**

(43) Veröffentlichungstag der Anmeldung:
**01.04.87 Patentblatt 87/14**

(45) Bekanntmachung des Hinweises auf die Patenterteilung:
**13.09.89 Patentblatt 89/37**

(84) Benannte Vertragsstaaten:
**AT BE CH DE FR GB IT LI NL SE**

(56) Entgegenhaltungen:
**EP-A-0 036 158**
**WO-A-79/00865**
**DE-A-836 839**
**FR-A-2 187 284**
**US-A-2 448 846**
**US-A-4 421 110**

(73) Patentinhaber: **STÖCKHERT, Heinz, Weiherweg 4,
D-8775 Partenstein (DE)**
Patentinhaber: **KONRAD, Paul, Hauptstrasse 4,
D-6991 Igersheim- Simmringen (DE)**

(72) Erfinder: **STÖCKHERT, Heinz, Weiherweg 4, D-8775
Partenstein (DE)**
Erfinder: **KONRAD, Paul, Hauptstrasse 4, D-6991
Igersheim- Simmringen (DE)**

(74) Vertreter: **Pöhner, Wilfried Anton, Dr.,
Kaiserstrasse 27 Postfach 63 23, D-8700 Würzburg
1 (DE)**

EP 0 215 858 B1

## Beschreibung

Die Erfindung betrifft eine Rückenstütze zur physikalischen Therapie von Erkrankungen der Wirbelsäule entsprechend dem Oberbegriff des Anspruches 1.

Eine derartige Vorrichtung ist aus der US-A-4 421 110 bekannt. Bereits die dort gezeigten und als Massageelemente wirkenden knopfartigen Körper sind in zwei parallel zueinander verlaufenden Geraden angeordnet, deren Abstand gleich der Breite eines Wirbelkörpers ist, wobei der Abstand benachbarten geometrischen Körper einer Reihe etwa die Länge eines Wirbelkörpers beträgt. Die Massage erfolgt über die gesamte Länge der Wirbelsäule gesehen im wesentlichen gleichmäßig.

Hiervon ausgehend hat sich die Erfindung die Weiterentwicklung derartiger Rückenstützen zur physikalischen Therapie der der Wirbelsäule dahingehend zur Aufgabe gemacht, daß besonders häufig vorkommenden Erkrankungen und Gefährdungen auf optimale Weise entgegengearbeitet wird.

Gelöst wird diese Aufgabe erfindungsgemäß dadurch, daß im Bereich der Lende oder des Beckens senkrecht zu den Geraden aufgereihte zweite geometrische Körper angebracht sind, die vorzugsweise niedriger als die ersten Körper sind.

Die angebrachten geometrischen Körper weisen nach außen zeigende Spitzen oder Kanten auf, die so angeordnet sind, daß die geometrischen Körper beidseitig an der Wirbelsäule anzuliegen kommen, demzufolge das Gewicht des Körpers unmittelbar an der Wirbelsäule und nicht wie üblich durch die außerhalb liegenden und durch die Rippen definierten Bereiche abgestützt wird. Da sich im Bereich der Lendenwirbel, also zwischen Brustkorb und Becken keine Rippen befinden, aber gerade in jenen Bereichen besonders häufig Schäden der Wirbelsäule und ein Verschleiß der Bandscheiben gegeben ist, finden sich zusätzliche geometrische Körper senkrecht zu den Geraden, hier vorzugsweise niedriger angeordnet. Die zusätzlichen Massageeffekte bringen Linderung entsprechender Nervenschmerzen und Ischiaserkrankungen. Durch den punktuellen Kontakt des Patienten und dessen unwillkürlichen Bewegungen entstehen Wechselkompressionen, die eine Förderung der Durchblutung und in Folge davon eine Stärkung der entsprechenden Muskelbereiche und eine Entspannung mit sich bringt. Darüberhinaus erhält man zusätzlich eine Entlastung des Beckens oder des Lendenwirbelbereiches und es wird das insbesondere bei sitzender Haltung häufig vorkommende Abkippen der Lendenwirbelsäule und die dadurch verursachten Schmerzen verhindert. Bei natürlicher Haltung liegt die Wirbelsäule tiefer im Körper als der Bereich des Beckens, weshalb es von Vorteil ist, wenn die Höhe der geometrischen Körper im Bereich des Beckens oder der Lende für eine anatomisch richtige Abstützung niedriger gewählt sind.

Zur baulichen Gestaltung der geometrischen Körper selbst bestehen unterschiedliche Möglichkeiten. So können sie beispielsweise als Vollkörper ausgebildet oder aber zumindest zu einem gewissen Teil hohl ausgeformt sein. Die geometrischen Körper sind mit der Unterlage durch Aufschweißen, Aufvulkanisieren oder Aufnähen verbunden oder mit dieser einstückig ausgebildet.

In einer bevorzugten Ausgestaltung ist die Oberfläche der gesamten Rückenstütze mit einem elastischen Material, wie z. B Stoff, überzogen. Man erhält eine homogene, leicht reinigbare und auch ästhetisch ansprechende Oberfläche. Die Funktion wird durch die Elastizität des verwendeten Materials nicht beeinträchtigt.

Die Form der Rückenstütze und insbesondere auch der Unterlage ist hierbei im Rahmen der Erfindung grundsätzlich beliebig, denn sie kann sowohl die Form einer Schale, insbes. eines Schalensitzes, einer Matratze oder aber lediglich einer Matratzenauflage besitzen.

In einer besonders bevorzugten Ausführungsform sind die geometrischen Körper mit Austrittsöffnungen und mit Schläuchen zur Versorgung mit Luft, Wasser o.dgl. versehen. Durch Beaufschlagung mit kühlem oder warmen Medium läßt sich eine Abkühlung oder Erwärmung bettlägriger Patienten und im Gefolge davon eine Anregung der Durchblutung vornehmen. Ein Unterstützung und Intensivierung der physikalischen Therapie ist die Folge. Ähnliches trifft auch zu, wenn Unterlagen und/oder geometrische Körper und damit die gesamte Rückenstütze beheizbar sind.

Schließlich ist noch das Anbringen von Befestigungsvorrichtungen von Vorteil, die als elastische Befestigungsbänder, Stülpüberzüge o.dgl. zur Anbringung auf einer Unterlage in Form einer Matratze, einer Stuhllehne, eines Autositzes, verwendet werden können.

Weitere Einzelheiten, Merkmale und Vorteile der Erfindung lassen sich dem nachfolgenden Beschreibungsteil entnehmen, in dem an Hand der Zeichnung ein Ausführungsbeispiel näher erläutert wird. Es zeigt in schematischer Darstellung:

Figur 1　eine Draufsicht auf eine erfindungsgemäße Rückenstütze,

Figur 2　ein Schnitt entlang der Linie I - I in Figur 1

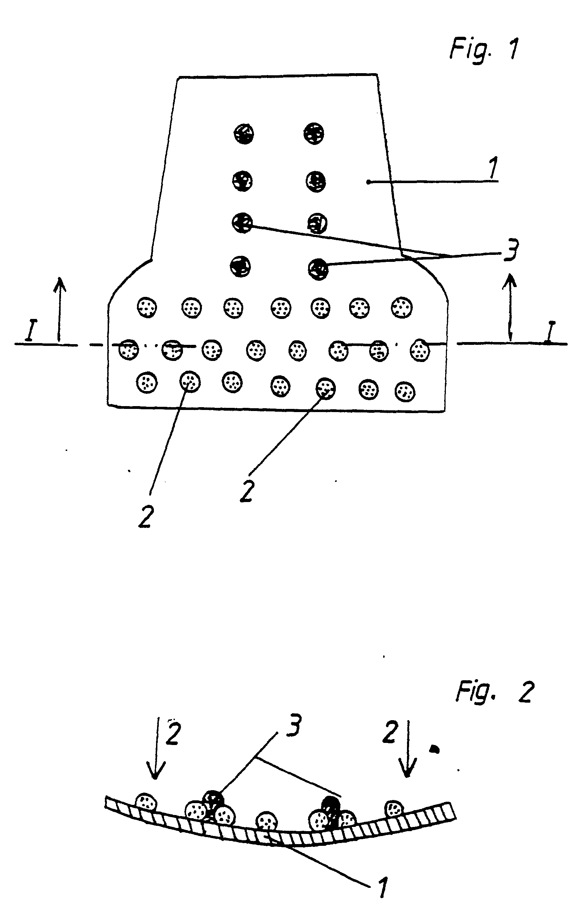

Figur 1 stellt eine erfindungsgemäße Rückenstütze dar, die in ihrem grundsätzlichen Aufbau aus einer Unterlage 1 und aus darauf angeordneten geometrischen Körpern 2, 3 bestehen. Die mit der Nummer 3 bezeichneten geometrischen Körper sind hierbei in zwei Geraden parallel zueinander und in entsprechendem Abstand angeordnet, so daß die Wirbelsäule des Benutzers hierdurch eine Abstützung erfährt. Ohne Einschränkung der Allgemeinheit sind darunter und etwa senkrecht hierzu weitere geometrische Körper 2 befestigt, deren Aufgabe in der Abstützung des Lenden- oder Beckenbereiches liegt. Die Form der Unterlage 1 ist so gewählt, daß die Rückenstütze als Lehne eines Stuhles oder eines Kraftfahrzeugsitzes verwendbar ist.

In Figur 2 ist ein Schnitt entsprechend der Linie I - I in Figur 1 wiedergegeben, aus der sich gut erkennen läßt, daß die in vertikaler Richtung verlaufenden geometrischen Körper 3 von größerer Höhe als die im Lenden- und Beckenbereich sitzenden geometrischen Körper 2 sind. Hierdurch wird der Tatsache Rechnung getragen, daß die Wirbel im Vergleich zum Becken weiter im Inneren des Körpers verlaufen, so daß eine anatomisch korrekte Abstützung vorgenommen werden kann. Aus Figur 2 ist gut erkennbar, daß die geometrischen Körper 2, 3 im dargestellten Ausführungsbeispiel etwa Halbkugeln sind. Die Unterlage 1 ist nach Art eines Schalensitzes gekrümmt.

Im Ergebnis stellt die Erfindung eine Rückenstütze zur Verfügung, mit deren Hilfe eine Stabilisierung der einzelnen Wirbelkörper, eine Steigerung und Förderung der Durchblutung und folglich auch eine Kräftigung der entsprechenden Muskulatur erreicht werden kann.

**Patentansprüche**

1. Rückenstütze zur physikalischen Therapie von Erkrankungen der Wirbelsäule bestehend aus einer Unterlage (1), auf die erste geometrische Körper (3) in Form von Pyramiden und/oder Kegel und/oder Zylindern und/oder Keilen und/oder Halbkugeln odgl. aufgebracht sind, die wenigstens zwei in Längsrichtung symmetrisch zur Mittelachse und parallel zueinander verlaufende Geraden bilden, deren Abstand etwa die Breite eines Wirbelkörpers ist und bei denen der Abstand benachbarter geometrischer Körper (3) etwa die Länge eines Wirbelkörpers ist,
dadurch gekennzeichnet,
daß im Bereich der Lende oder des Beckens senkrecht zu den Geraden aufgereihte zweite geometrische Körper (2) eingebracht sind, die vorzugsweise niedriger als die ersten Körper sind.

2. Rückenstütze nach Anspruch 1,
dadurch gekennzeichnet,
daß die geometrischen Körper (2, 3) zumindest außenseitig elastisch ausgebildet sind.

3. Rückenstütze nach Anspruch 2,
dadurch gekennzeichnet
daß die Elastizität der geometrischen Körper (2, 3) so eingestellt ist, daß sie bei den auftretenden Belastungen nur den äußeren Bereichen deformiert werden.

4. Rückenstütze nach einem der Ansprüche 1 bis 3,
dadurch gekennzeichnet
daß die geometrischen Körper (2, 3) zumindest teilweise hohl sind.

5. Rückenstütze nach einem der Ansprüche 1 bis 4,
dadurch gekennzeichnet,
daß die geometrischen Körper (2, 3) aufgeschweißt, aufvulkanisiert oder aufgenäht sind.

6. Rückenstütze nach einem der Ansprüche 1 bis 5,
dadurch gekennzeichnet,
daß die Oberfläche mit elastischem Material, wie z. B. Stoff, überzogen ist.

7. Rückenstütze nach einem der Ansprüche 1 bis 6,
dadurch gekennzeichnet
daß die Rückenstütze die Form eines Sitzes (Schalensitzes), einer Matratze oder einer Matratzenauflage besitzt.

8. Rückenstütze nach einem der Ansprüche 1 bis 7,
dadurch gekennzeichnet
daß die geometrischen Körper (2, 3) Austrittsöffnungen aufweisen und mit Schläuchen zur Versorgung mit Luft, Wasser o.dgl. versehen sind.

9. Rückenstütze nach einem der Ansprüche 1 bis 8, gekennzeichnet durch Beheizbarkeit.

10. Rückenstütze nach einem der Ansprüche 1 bis 9,
dadurch gekennzeichnet,
daß die Rückenstütze mit Befestigungsvorrichtungen versehen ist.

**Claims**

1. Back support for physical therapy of illnesses of the final column comprising a support (1), on which the first geometrical body (3) in the form of a pyramid and/or cone and/or cylinders and/or wedges and/or hemispheres or similar are disposed, which form at least two straight lines extending symmetrically to the central axis and parallel to each other, the distance between which is approximately the width of a vertebra and in which the distance between adjacent geometrical bodies (3) is approximately the length of a vertebra
wherein in the region of the loin or pelvis vertically to the straight lines a second row of geometrical bodies (2) is disposed, which are preferably lower than said first bodies.

2. Back support according to claim 1
wherein said geometrical bodies (2, 3) are elastic at least externally.

3. Back support according to claim 2
wherein the elasticity of said geometrical bodies (2, 3) is adjusted such that when subjected to loads only the outer regions are deformed.

4. Back support according to one of claims 1 to 3
wherein said geometrical bodies (2, 3) are at least partially hollow.

5. Back support according to one of claims 1 to 4
wherein said geometrical bodies (2, 3) are welded, vulcanization bonded or stitch bonded.

6. Back support according to one of claims 1 to 5 wherein the surface is covered with elastic material, e.g. fabric.

7. Back support according to one of claims 1 to 6 wherein the back support is in the form of a seat (bucket-seat), a mattress or a mattress cover.

8. Back support according to one of claims 1 to 7 wherein said geometrical bodies (2, 3) have outlet openings and are provided with hoses for supply with air, water or similar.

9. Back support according to one of claims 1 to 8 wherein said back support can be heated.

10. Back support according to one of claims 1 to 9 wherein said back support is provided with fastening devices.

**Revendications**

1. Un soutien dorsal pour la physico-thérapie des maladies de la colonne vertébrale qui consiste en un support (1) sur lequel sont fixés de premiers éléments géométriques (3) en forme de pyramides et (ou) de cônes et (ou) de cylindres et (ou) en forme de triangle et (ou) de demi-sphères, ou autres, formant au moins deux droites parallèles dans le sens de la longueur et symétriques a l'axe central. La distance entre ces deux droites étant environ égale à la largeur d'une vertèbre et la distance entre éléments géométriques voisins étant environ égale à la longueur d'une vertèbre. Ce soutien dorsal est caractérisé par de seconds éléments géométriques (2) alignés à la verticale des droites dans les régions lombaires et du bassin et présentant l'avantage d'être plus bas que les premiers éléments.

2. Un soutien dorsal d'après la revendication 1 et caractérisé par des éléments géométriques (2, 3) conçus en matière plastique au moins vers l'extérieur.

3. Un soutien dorsal d'après la revendication 2 caractérisé par le fait que l'élasticité des éléments géométriques (2, 3) est étudiée de telle manière que les éléments géométriques ne se déforment que vers l'extérieur lors de pressions.

4. Un soutien dorsal d'après les revendications 1 à 3 caractérisé par le fait que les éléments géométriques (2, 3) sont creux, au moins en partie.

5. Un soutien dorsal d'après les revendications 1 à 4 caractérisé par le fait que les éléments géométriques (2, 3) sont soudés, vulcanisés ou cousus sur le support.

6. Un soutien dorsal d'après les revendications 1 à 5 caractérisé par le fait que la surface est recouverte d'une matière élastique, de tissu par exemple.

7. Un soutien dorsal d'après les revendications 1 à 6 caractérisé par le fait que ce soutien possède la forme d'un siège (siège coquille), d'un matelas ou d'un couvre-matelas.

8. Un soutien dorsal d'après les revendications 1 à 7 caractérisé par le fait que les éléments géométriques sont pourvus d'ouvertures permettant grâce à des tuyaux l'alimentation en air, eau ou autres.

9. Un soutien dorsal d'après les revendications 1 à 8 caractérisé par le fait qu'il est chauffant.

10. Un soutien dorsal d'après les revendications 1 à 9 muni d'un système de fixation.

*Fig. 1*

*Fig. 2*